(19)

Europäisches Patentamt
European Patent Office
Office européen des brevets

(11) **EP 2 991 690 B1**

(12) **EUROPEAN PATENT SPECIFICATION**

(45) Date of publication and mention of the grant of the patent:
**08.03.2017 Bulletin 2017/10**

(21) Application number: **14727923.6**

(22) Date of filing: **23.04.2014**

(51) Int Cl.:
***A61L 2/22*** *(2006.01)*

(86) International application number:
**PCT/IB2014/060943**

(87) International publication number:
**WO 2014/177976 (06.11.2014 Gazette 2014/45)**

(54) **METHOD AND DEVICE FOR DISINFECTING AND PERFUMING OF SPACES AND SURFACES**

VERFAHREN UND VORRICHTUNG ZUR DESINFEKTION UND PARFÜMIERUNG VON RÄUMEN UND OBERFLÄCHEN

PROCÉDÉ DE DIFFUSION ET DISPOSITIF DE DÉSINFECTION D'ESPACES ET DE SURFACES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2013 IT BO20130193**

(43) Date of publication of application:
**09.03.2016 Bulletin 2016/10**

(73) Proprietor: **99 Holding S.A.R.L**
**1449 Luxembourg (LU)**

(72) Inventors:
- **MALAGUTI SIMONI, Gian, Luca**
  **98000 Monaco (MC)**
- **SICCARDO, Giovanni**
  **I-24122 Bergamo (IT)**

(74) Representative: **Firmati, Leonardo**
**Bugnion S.p.A.**
**Via di Corticella, 87**
**40128 Bologna (IT)**

(56) References cited:
**WO-A1-00/50320       US-A1- 2010 326 117**
**US-A1- 2011 309 534**

## Description

### Technical field

This invention relates to a diffuser method and device for the disinfection of spaces and surfaces.

### Background art

The invention applies in particular to the disinfection of rooms and the surfaces contained therein, by the nebulising and diffusion of disinfectant solutions.

One of the age-old problems affecting public structures has always been that linked to the risk of their visitors catching infections. Amongst the public area that potentially suffer from this problem we can list, amongst others: schools, public offices, hotels, restaurants, trains, aircraft and hospitals, the latter of particular interest given the high number of users and their particular state of health.

In the hospital context it is possible to identify at least two types of patients/operators:

- in-patients, possibly "run-down" (immunosupressed, the elderly with multiple pathologies, premature babies, malnourished individuals), the organisms of whom do not have the capacity to respond adequately to bacterial attacks;
- patients who undergo surgery, for whom it has been demonstrated that 10 CFU/m$^3$ is sufficient to cause a serious arthropathy, where CFU/m$^3$ is the unit of measurement of the volumetric "colony-forming unit (CFU)" , that is, the concentration of the CFUs per unit of volume, whilst CFU/m$^2$ is the unit of measurement of the surface CFUs.

In light of this, the prior art proposes disinfectant solutions which are able to instantaneously attack all the organic substances with which they enter into contact, destroying viruses, bacteria, spores, fungi and biofilm present in the air and on the surfaces.

These disinfectant solutions can be diffused in the room by devices which are able to transform them from the liquid state to that of dry steam which, after having disinfected the air, deposits on all the surfaces, attacking the micro-organisms present.

Although the effectiveness of the diffuser systems has been widely demonstrated, and have the advantage of a potential repetition of the treatment and, consequently, the results of the disinfection, the repetition is, however, guaranteed by the capacity of the diffuser system to perform a correct disinfection, and certify that it has been performed, in a given environment. More specifically, to guarantee and certify that:

- the desired room has been sanitised;
- the specified sanitising solution has been used;
- the solution has been dispensed in a continuous manner during the entire dispensing;
- the planned quantity of solution has been dispensed.

In effect, the systems for the disinfection of spaces and surfaces which are currently available on the market typically consist of a single diffuser device (or nebuliser), which can be programmed for use and be programmed by an operator for an operation of predetermined duration, which does not allow the correctness of the sanitisation treatment of a specific room to be guaranteed and certified.

Prior art diffuser devices are disclosed in documents US 2010/326117 and WO 00/50320.

### Disclosure of the invention

The aim of this invention is to provide a diffuser method and device for the disinfection of spaces and surfaces which can overcome the above-mentioned drawbacks of the prior art.

More specifically, the aim of this invention is to provide a diffuser method and device for the disinfection of spaces and surfaces which allows the operator to immediately check the correct sanitisation treatment of the room.

Moreover, the aim of this invention is to provide an automatic and safe method and device for the disinfection of spaces and surfaces, which guarantees the accuracy of the disinfection.

These aims are achieved by a diffuser method and device for the disinfection of spaces and surfaces having the features described in one or more of the claims from 1 to 10.

More specifically, these aims are achieved by a method for the disinfection of spaces and surfaces, preferably but not solely implemented by the diffuser device according to claim 1.

The method comprises preparing a diffuser device equipped with at least one tank containing a liquid solution and at least nebulising means associated with the tank and designed for drawing out the liquid solution from the tank spraying it in the space.

Thus, the liquid solution is nebulised in the room in a predetermined quantity for an interval of time, following which, immediately or preferably after a (further) predetermined interval of time, a predetermined quantity of a liquid substance, different from the liquid solution and having a predetermined fragrance is then nebulised in the room, representing an end of the treatment.

It should be noted that it is also possible to use a further liquid substance with a different fragrance, which can be sprayed in the room in the case of a malfunction of the diffuser device or problems linked with the effectiveness of the liquid substance.

Advantageously, in this way not only the sensorial check of the effectiveness of the treatment would be allowed, but also a corresponding sensorial control (again, olfactory) of an error in the treatment.

As mentioned, the invention is also correlated with a diffuser device for a system for the disinfection of spaces and surfaces equipped with a tank containing a liquid solution, a container of a liquid substance, different from the liquid solution and having a predetermined fragrance, nebulising means in fluid connection with the tank and the container and configured for drawing out the liquid solution from the tank and the liquid substance from the container spraying them in the space and at least one control unit programmed for controlling the nebulising means in such a way as to draw out selectively from the tank or from the container as a function, respectively, of a first signal representing the start of a sanitisation and of a second signal representing the end of a sanitising or the nebulising. Advantageously, in this way the checking of the effectiveness of the treatment is immediate from the sensorial point of view, avoiding longer and more complicated controls by the operator.

It should be noted that, alternatively or in addition, there could also be the activation of sound (or luminous) emission means which complete or, possibly, replace the nebulising of the liquid substance (or fragrance) allowing a sensorial check of an auditory type.

Preferably, the diffuser device comprises sensor means associated with the tank and the control unit and configured for detecting a quantity correlated with the quantity of liquid solution dispensed by the nebulising means and to send a signal representing the quantity to the control unit.

In light of this, the control unit is programmed for issuing a command to the nebulising means for drawing out the liquid substance from the container after receiving, from the sensor means, a signal indicating that the threshold value of the quantity of liquid solution to be dispensed has been exceeded.

Preferably, the sensor means comprise at least one weight sensor associated with the tank, in such a way that the control unit can calculate the variation in the quantity of liquid solution in the tank as a function of a variation of the weight of the tank.

Other types of sensors of the quantity of liquid solution dispensed and/or available, such as, for example, ultrasonic volume sensors or capacitive sensors, can be considered.

It should be noted the control unit is configured for calculating the threshold value of the quantity of liquid substance to be dispensed as a function of the cubic volume of the room to be treated.

Advantageously, in this way the dispensing of the sanitising liquid solution is automatic and certain, since it is backfed, thus increasing the efficiency and effectiveness of the device.

Preferably, the control unit can be associated with a plate (electronic) containing a plurality of identification data of a room to be treated in such a way as to receive from the plate a signal representing the identification data of the room.

In this way, the calculation of the predetermined quantity of liquid solution to be dispensed is also automatic, overcoming to problems linked to a manual setting by the operator, which by its very nature is subject to errors.

## Brief description of the drawings

These features of the invention will become more apparent from the following detailed description of a preferred, non-limiting embodiment of it, illustrated by way of example in the accompanying drawings, in which:

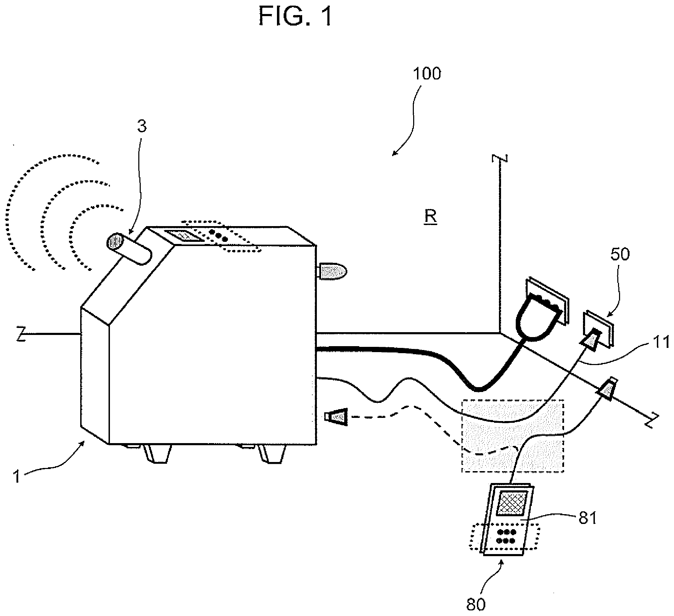

- Figure 1 shows an assembly view of the preferred implementation of a system for disinfecting spaces and surfaces comprising a diffuser device according to this invention;

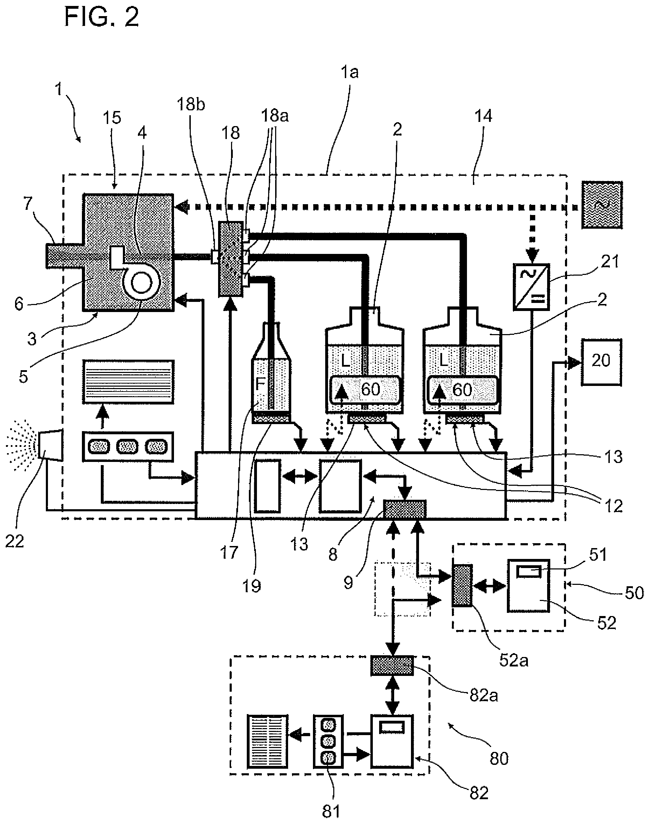

- Figure 2 shows a schematic and functional view of the system of Figure 1;
- Figure 3 shows a first embodiment of the communication channel between the diffuser device and the plate;
- Figure 4 shows a second embodiment of the communication channel between the diffuser device and the plate;
- Figures 5a, 5b and 5c show a schematic and functional view of the means for identifying the liquid solution inside the tank;
- Figure 6 shows a series of curves of the function Vout = Vout (Weight), produced by a weight sensor operating on the tank.

## Detailed description of the preferred embodiments of the invention

With reference to the accompanying drawings, the numeral 1 denotes a diffuser device for a system 100 for the disinfection of spaces and surfaces according to this invention.

The diffuser device 1 is preferably inserted inside a system 100 for the disinfection of spaces and surfaces comprising at least one plate 50 containing a plurality of identification data of a room "R" to be treated. More specifically, the plate 50 is equipped with a memory 51 containing a plurality of identification data of the room "R". The term "identification data" is used to comprise one or more of the following data:

- property;
- building and floor;
- room number;
- cubic volume;
- purpose.

Preferably, the plate 50 comprises a processor 52 associated with the memory 51 and programmable by an operator for entering the identification data of the "R".

Even more preferably, the processor 52 is equipped with a transmission module 52a connectable to the diffuser device 1 for sending to it the identification data of the room "R" to be treated.

Thus, the plate 50 comprises a processor 52 (more specifically a microcontroller), a memory 51 (of non-volatile type) and a transmission module 52a equipped with an interface circuit towards a two-way communication channel with the diffuser device 1.

The diffuser device 1 comprises a tank 2 containing a liquid solution "L" and nebulising means 3, associated with the tank 2 and designed to draw out the liquid solution "L" from the tank 2 spraying it in the space. Preferably, the nebulising means 3 comprises a turbine 4 connected to the tank 2 through an infeed pipe from which it receives, using a pump 5, the liquid solution "L" to be nebulised.

Downstream of the turbine 4 there is also a Venturi effect (or "cross flow") pneumatic nebuliser 6, which is in fluid connection with the turbine 4 to receive a flow of air at high pressure which flows around the infeed capillary tube of the liquid. At the outfeed of the capillary tube the liquid solution "L" entering is transformed into aerosol by the air and then diffused into the space by a disperser 7. Other types of nebulisers, different from those described in the preferred embodiment, may be considered.

According to this invention, the diffuser device 1 comprises a control unit 8 associated with the nebulising means 3 (and which can be preferably associated with the plate 50). In the preferred embodiment, the control unit 8 comprises at least one microprocessor.

The control unit 8 is set up to receive from the plate 50 (and in particular from the transmission module 52a) a signal representing identification data of the room "R" and configured for controlling the nebulising means 3 as a function of that signal, so as to a sanitise the room in an automatic manner.

Thus, the control unit 8 comprises a data reception module 9 connectable to the plate 50, that is, to the transmission module 52a, for receiving the signal representing the identification data of the room "R".

More specifically, the control unit 8 is programmed to communicate with the plate 50 continuously during the nebulising, that is, during the treatment. In other words, the control unit 8 is designed to interrupt the nebulising (that is, to command the stopping of the nebulising means 3) following a communication failure between the data reception module 9 and the plate 50 so as to guarantee that the diffuser system is not moved to another room whilst the sanitising process is in progress. Advantageously, this makes it possible to guarantee that the diffuser device 1 is not removed from the room whilst the treatment is in progress. More in detail, the data reception module 9 and the transmission module 52a define a communication channel (two-way) between the diffuser device 1 and the plate 50.

The two-way communication channel allows the diffuser device 1 to communicate with the plate 50 when the two reception 9 and transmission 52a modules are connected (that is, in communication with each other).

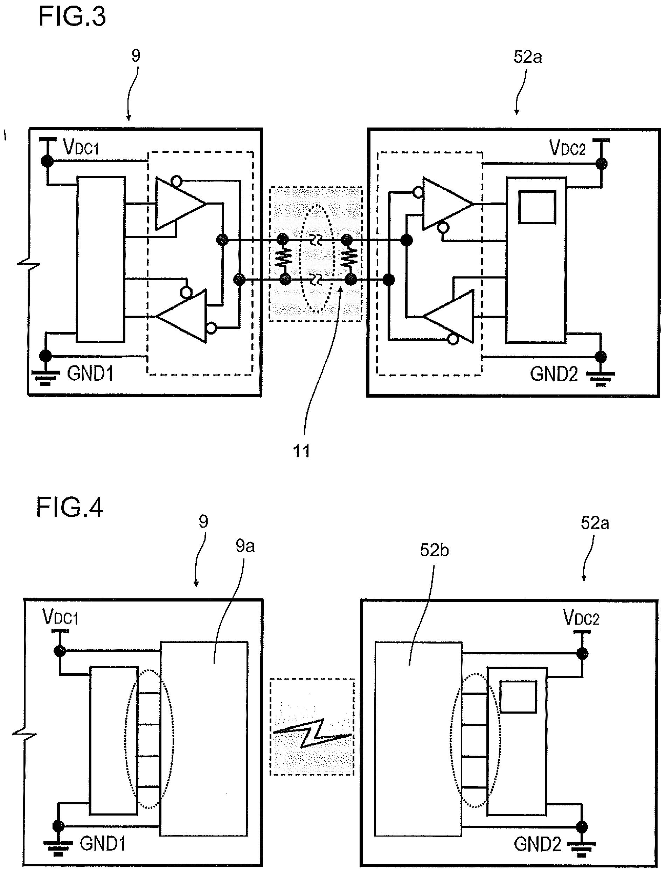

In a first embodiment (Figure 4) the communication channel is a wireless bus, such as Bluetooth or Bluetooth Low Energy (BLE).

Thus, the data reception module 9 of the control unit 8 of the diffuser device 1, communicates with the transmission module 52a of the processor 52 of the electronic plate 50 by wireless communication.

From the physical point of view, the two data reception 9 and transmission 52a modules are defined by Bluetooth interface modules 9a, 52b.

It should be noted that the control unit 8 and the processor 52 interface with the corresponding data reception 9 and transmission 52a modules using a plurality of status and control signals.

The adoption of other wireless, or short range, communication technology, such as, for example, CIR (Consumer Infra Red) may be assessed and, possibly, adopted.

**EP 2 991 690 B1**

In an alternative embodiment (Figure 3), the communication channel is defined by a serial bus. The communication allows the two-way exchange of data, in half duplex mode (one only transmitter active at a time), with a master-slave protocol, with the control unit 8 in the role of master and the processor 52 in the role of slave. From the physical point of view, the data reception module 9 and the transmission module 52a are defined by a transceiver (transmitter and receiver), in turn connected by cable 11. Preferably, the transceivers are equipped with a termination resistor on each of the two sides of the communication channel. On the side of the diffuser device 1, and of its control unit 8, there is both a transmitter and a receiver.

The above-mentioned transmitter sends on the cable 11 the data to be transmitted which it receives from the control unit 8 (together with a transmission enabling signal).

The above-mentioned receiver provides to the control unit 8 data read from the above-mentioned cable 11 (after receiving the transmission enabling signal provided by the control unit 8).

Similarly, on the side of the processor 52 it is possible to identify both the transmitter and the receiver.

Preferably, the control unit 8 is programmed for calculating a quantity of the liquid solution "L" to be introduced into the room for nebulising as a function of the signal representing identification data of the room "R" sent by the plate 50.

In order to check that this quantity is correct, the diffuser device 1 comprises sensors means 12 associated with the tank 2 and with the control unit 8 and configured to measure a quantity correlated with the quantity of liquid solution "L" dispensed from the nebulising means 3.

The sensor means are also configured for sending a signal representing the quantity to the control unit 8.

In turn, the control unit 8 is configured for calculating a threshold value of the quantity of liquid solution "L" to be dispensed as a function of the signal representing the identification data of the room "R" received from the plate 50 and for comparing the threshold value with the quantity of liquid solution "L" dispensed.

Moreover, the control unit 8 is configured to interrupt the nebulising by the nebulising means 3 (and thus the action of the turbine 4) when the quantity of liquid solution "L" dispensed equals or exceeds the threshold value.

Preferably, the sensor means 12 comprise at least one weight sensor 13 associated with the tank 2.

Thus, the control unit 8 is configured for calculating a variation of the quantity of liquid solution "L" in the tank 2 as a function of a variation of the weight of the tank 2.

More in detail, the tank 2 is positioned in a housing 14 made in a structure 1 a of the diffuser device 1.

At the base of the housing 14 there is the weight sensor 13, the output signals of which are connected with the control unit 8 so as to be read, determining the current weight of the tank 2 and, consequently, the amount of sanitising solution instantaneously present in the tank 2.

Due to its ease of use and low cost, the weight sensor 13 adopted in the preferred embodiment is of the FSR (Force Sensitive Resistor) type. Other types of weight sensors (e.g. load cell) may be considered.

In this circuit the resistance of the "RFORCE" sensor is represented by the variable resistance, connected to the first input of the operational amplifier. At the second input of the above-mentioned amplifier there is a reference voltage "VREF" applied by a divider. An output of the above-mentioned amplifier is backfed to the input by the feedback resistance (RFDBK) and a filter capacity. The value of the output voltage is given by the following formula:

$$VOUT = VREF \times (1 + (RFDBK\ /RFORCE))$$

An analogue-digital converter makes it possible to read the outfeed voltage value VOUT and correlate it with the weight of the tank 2.

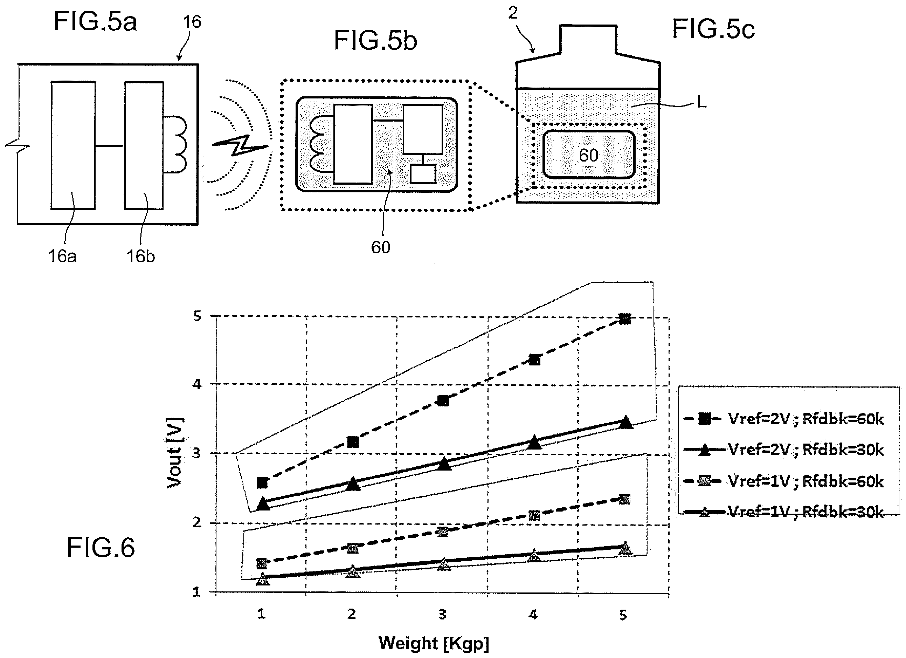

Figure 6 illustrates the trend of the function VOUT = VOUT (Weight) with the variation of the parameters VREF and RFDBK which determine the sensitivity (slope) and the capacity of the sensor.

Figure 6 shows the trend of the function VOUT = VOUT (Weight), that is, how the output signal VOUT varies with the variation of the independent Weight variable. The diagram illustrates four parametric curves varying the parameters VREF and RFDBK: the two curves show the trend of the function when VREF corresponds to 1V and RFDBK changes from 30 k$\Omega$ to 60 k$\Omega$.

The other two curves show the trend of the function when VREF corresponds to 2V and RFDBK changes from 30 k$\Omega$ to 60 k$\Omega$.

By comparing the two pairs of curves it may be noted that VREF determines the minimum weight that the sensor is capable of measuring. However, by fixing, for example, VREF = 2V it may be noted that RFDBK determines the inclination of the curve.

It is evident from the diagram that by varying the two parameters VREF and RFDBK it is possible to vary the capacity and the sensitivity of the sensor, thereby adapting it to the different requirements of the weight sensors 13.

Preferably, also, the nebulising means 3 comprise means 15 for adjusting the flow rate of the nebulised liquid solution "L" , also associated with the control unit 8.

The control unit 8 is programmed for sending to the adjustment unit 15 a signal representing the flow rate of liquid solution "L" to be nebulised as a function of the consumption of liquid solution "L" in the tank 2 measured by the sensor means 12.

More specifically, at the start of the treatment the control unit 8 acquires the weight of the tank 2, assuming that the latter is the container in use, and the data of the room "R" to be treated using the plate 50. Knowing the type of room to be processed, for example, in the hospital context, patients ward or operating room, and the volume to be treated, the control unit 8 (that is, the microprocessor) can determine the quantity of sanitising solution to be dispensed and the time necessary. After checking that the tank 2 contains a total quantity of sanitising liquid solution "L" sufficient to complete the dispensing, the control unit 8 starts the treatment activating the turbine 4 of the nebulising means 3 the pump 5, with the average flow rate necessary to complete the treatment in the calculated time. At predetermined intervals of time, the control unit 8, measuring the quantity of solution dispensed in the interval of time and comparing a deviation of the curve of actual consumption (quantity dispensed divided by the time) with the calculated theoretical consumption, determines the correction which must be made to the flow rate of the pump 5 so that the actual flow rate is aligned with the theoretical flow rate.

Advantageously, the pump 5 also allows faults in the system for feeding the nebulising means 3 to be detected. In effect, the control unit 8 by operating together with the sensor means 12 of a quantity correlated with the quantity of liquid solution "L" dispensed, can also check that the consumption of liquid solution "L" is consistent with the flow rate of the pump 5. The nebulising can be interrupted and an error message displayed and recorded, if consumption is greater or less than the set one.

The control unit 8 is also programmed to check (at preset time intervals) that the connection with the plate 50 is enabled, to guarantee that the sanitising is being performed in the preset room and that the diffuser system has not been moved to another room.

The end of the treatment (that is, of the nebulising) will be determined by the reaching the dispensing of the quantity of solution provided, favouring this parameter with respect to time of the treatment.

In other words, if the threshold value of sanitising liquid solution "L" dispensed is reached before a preset duration of the planned treatment, the control unit 8 still sends to the nebulising means a signal representing an interruption of the treatment, stopping it.

In the preferred embodiment, the diffuser device 1 comprises two tanks 2. In the same way, the diffuser device 1 comprises two weight sensors 13, each associated with a respective tank 2.

Preferably, also, the tanks 2 are removable and can be replaced. To allow the effectiveness of the contents to be checked, the diffuser device 1 comprises recognition means 16 which can be associated with each tank 2 and configured for identifying the liquid solution "L" contained in the tank 2. These recognition means 16 are associated with the control unit 8.

More specifically, the recognition means 16 comprise a reading unit 16a which can be associated with a label 60 applied to the tank 2 for reading one or more identification data of the liquid solution "L" contained in the label 60.

Preferably, also, the label 60 is rewritable. In this regard, the recognition means 16 also comprise a writing module 16b designed for modifying the identification data of the liquid solution "L" as a function of the consumption of the liquid solution measured by the sensor means 12. These recognition means 16 operate on both the tanks 2.

Thus, each tank 2 is equipped with a relative electronic label 60, containing all the production data of the respective sanitising solution (manufacturer, type of sanitising fluid, date and place of production, expiry date, etc) in addition to the weight of the above-mentioned solution contained therein.

These labels 60 are irremovably attached to the above-mentioned tank 2 and contain readable and writable information.

Preferably, the labels 60 are configured to communicate with the recognition means 16 using two-way wireless and short-range channels. The electronic labels 60 contemplated in the preferred embodiment are RFID (Radio Frequency IDentification) TAGS.

Other embodiments which are able to contain readable and writable information, non-removable from the tanks 2, that is to say, that attempts to remove cause permanent damage, and which are able to communicate on two-way channels (read and write) with a control card, can be considered.

Preferably, the memory (non-volatile) of the electronic label 60 records (preferably in a coded manner) not only the identification data of the above-mentioned solution, but also the weight of the tank 2 from which, by subtracting the tare weight of the container, it is possible to obtain the weight of the sanitising solution available in the above-mentioned tank 2. The previous data are recorded in the above-mentioned memory, together with a redundancy code, to protect the integrity of the information recorded.

According to the invention, the diffuser device 1 also comprises a container 17 of a liquid substance "F", having a predetermined fragrance.

The container 17 is placed in fluid connection with the nebulising means 3. The control unit 8 is programmed for drawing out the liquid substance "F" from the container 17 and nebulising it in the room "R" after receiving, from the sensor means 2, a signal indicating that the threshold value of the quantity of liquid solution "L" to be dispensed has been exceeded.

More specifically, the control unit 8 is programmed for controlling the nebulising means 3 in such a way as to draw out selectively from the tank 2 or from the container 17 as a function, respectively, of a first signal representing the start of a sanitisation and of a second signal representing the end of the nebulising.

Preferably, the control unit 8 is programmed for measuring a (further) predetermined interval of time between the end of nebulising and the dispensing of the liquid substance "F" so as to allow the complete removal of the bacteria by the sanitising liquid solution "L" nebulised during the treatment.

In other words, the second signal is emitted after a (further) predetermined period of time following the end of the nebulising/sanitising.

Preferably, the nebulising means 3 comprise a solenoid valve 18 with at least two (preferably three) paths having a plurality of inlets 18a, associated with the tank 2 (or the tanks 2) and the container 17.

The control unit 8 is configured for opening the passage of one of the inlets towards a single outlet 18b as a function of the signal received from the control unit 8.

Thus, upon the positive completion of the nebulising, the control unit 8 (that is, the microprocessor) of the diffuser commands a dispensing of the liquid substance "F" and, therefore, the nebulising of the respective fragrance for a time needed by the user to make an olfactory check of the positive completion of the sanitising of the room.

As specified above, in the preferred embodiment, the control unit 8 is programmed to await the passing of the further predetermined interval of time, following the positive end of the nebulising, before issuing a command to the nebulising means for drawing out the liquid substance "F" from the container 17.

More specifically, the solenoid valve 18 receives the sanitising liquid solution "L" from the tanks 2 through the pipes, respectively, or the liquid substance "F" (that is, the fragrance) from the container 17. Under the control of the signals generated by the control unit 8, the solenoid valve is controlled in such a way as to allow the flow of only one of the liquids, directing it to the turbine 4 of the nebulising means 3. Alternatively, however, other solutions for selecting the liquid to be nebulised, different from that described in this preferred embodiment, may be considered.

It should be noted that, similarly to the tanks 2, a further sensor 19, which is able to determine the weight of the fragrance present in the container 17, is installed at the base of the housing of the container 17. The above-mentioned further weight sensor 19 is connected to the control unit 8 of the diffuser device 1, by an interface circuit, similar to that adopted for determining the weight of the sanitising solution. The control unit 8 is thus able to automatically determine the quantity of liquid substance "F" (that is, fragrance) dispensed at the end of the treatment, as well as the consumption of the fragrance.

Preferably, the control unit is configured for calculating a predetermined quantity of liquid substance "F" to be dispensed as a function of the cubic volume of the room "R" to be treated.

Thus, the predetermined quantity of liquid substance "F" is calculated as a function of the identification data of the room "R" received from the plate 50.

It should be noted that the diffuser device 1 comprises a memory 20 (non-volatile and removable) associated with the control unit 8 and designed to receive from it, during or at the end of each nebulising, a plurality of data correlated with:

- the room treated and/or
- the quantity of liquid solution "L" nebulised and/or
- the quantity of liquid solution "L" remaining in the tank 2 and/or
- the date and time of nebulising and/or
- the duration of the nebulising.

Preferably, the control unit 8 is configured for periodically updating the memory 20 when reaching or exceeding the predetermined quantity of liquid substance "F" (that is, fragrance) dispensed.

In that way, as well as the sequence of treatments performed in each room "R", it is possible to trace in a continuous and/or complete manner the sequence of treatments performed by the diffuser device 1. Preferably, moreover, the diffuser device comprises sound emission means 22 associated with the control unit 8, wherein the control unit 8 is configured for activating the sound emission means 22 after reaching or exceeding a threshold value of the quantity of liquid substance "F" to be dispensed.

It should be noted that the diffuser device 1 may be powered both by a battery and by connection to the electricity network.

Preferably, in this regard, the diffuser device 1 is equipped with an AC/DC power supply module 21 configured for receiving the AC voltage (by a cable connected to the wall-mounted power supply socket).

The DC voltage, converted from the AC voltage and reduced in voltage, is supplied to the control unit 8 by means of an internal DC power supply cable. The other functional modules that require a DC supply voltage receive it from the above-mentioned control unit. The functional modules that require an AC supply voltage receive it directly from the internal power supply cable.

With reference to the entire system 100 for disinfecting rooms and surfaces, according to the invention, it comprises at least one control device 80 which can be used by an operator and connectable to the diffuser device 1.

The control device 80 comprises an interface module 81 which can be used by the operator and at least one processing module 82 connectable to the memory 20 of the diffuser device 1 for picking up a plurality of data correlated with each nebulising.

Similarly, the control device 80 is connectable to the plate 50.

More specifically, the processing module 82 can be connected to the processor 52 of the plate 50 to enable the operator to enter the identification data of the room "R" to be treated.

Moreover, the control device 80 is connectable to the memory 51 of the plate 50 to pick up the data relating to the treatments performed in the respective room "R" thereby allowing the quality and the frequency of treatments to be traced and kept under control.

Thus, the control device 80 is configured for allowing an operator to communicate in a two-way fashion both with the processor 52 of the above-mentioned electronic plate 50 and with the control unit 8 (or microprocessor 8a) of the above-mentioned diffuser device 1.

More specifically, the control device 80 is programmed for allowing an operator to initialise the memory 51 (non-volatile) of the processor 52 of the above-mentioned plate 50 with all the data relative to the room "R" in which it has been installed.

Moreover, the control device 80 is configured for collecting the data recorded in the memory 20 (non-volatile) of the above-mentioned microprocessor 8a of the diffuser device 1.

Preferably, the control device 80 will be made available only to authorised operators.

In this regard, the processing module 82 of the control device 80 is programmed to set up an identification procedure. More specifically, the module 82 is programmed for requesting identification data from the operator and comparing it with corresponding data held in the memory of the plate 51 or of the diffuser device 20, allowing access only if the data provided by the operator corresponds with data in the respective memory 20, 51.

Preferably, however, the system 100 is configured for transferring the data in coded form and to make the data readable only when the above-mentioned control device 80 is connected to an apparatus outside the system, such as a personal computer, where the data may be analysed, printed and archived.

Thus, the system 100 according to this invention provides a method for sanitising rooms and surfaces which is highly efficient and traceable at every step.

The method for the disinfection of spaces and surfaces comprises firstly preparing a diffuser device 1 equipped with a tank 2 containing a liquid solution "L" and nebulising means 3 associated with the tank 2 and designed for drawing out the liquid solution "L" from the tank 2 spraying it in the space.

Preferably, it comprises providing a plate 50, as described above, inside a room to be sanitised.

There is also a step of setting up identification data of the room, preferably using the control device 80.

In the preferred embodiment, a plurality of plates 50 is positioned in a corresponding plurality of rooms "R" to be sanitised and, preferably, each of them is set up with the identification data of the respective room.

Then, at the time of sanitising, a diffuser device 1 is located inside the room "R" to be treated.

The diffuser device 1 is placed in communication with the plate 50 for detecting the above-mentioned identification data.

Preferably, the control unit 8 of the diffuser device 1 is put in communication (through the two-way channel described above) with the processor 52 of the plate 50.

Thus, a predetermined quantity of liquid solution "L" is nebulised in the room for an interval of time.

It should be noted that both the interval of time and the flow of sanitising liquid solution "L" are a function of the identification data measured by the plate 50.

Preferably, during the nebulising there is a step of controlling the consumption of the liquid solution "L" by means of a comparison between the consumption measured and an estimated value as a function of the identification data.

For this purpose, even though different ultrasound or capacitive volumetric sensors can be considered, preferably the consumption is measured in relation to the variation of weight of the tank 2 of liquid solution "L", more preferably by the use of sensor means 12 described previously.

When a threshold value of liquid solution dispensed (that is, consumed) is reached or exceeded and/or at the end of the estimated time interval, the nebulising is interrupted.

Preferably, the quantity of liquid solution "L" is a predominant parameter relative to the duration of the treatment, as the latter is subordinate to the reaching of the above-mentioned threshold value.

As already mentioned, during the nebulising the diffuser device 1 (at regular intervals of time) contacts the plate 50, checking that the communication between them is active and that therefore the above-mentioned diffuser has not been moved from the room "R" to be treated.

According to the invention, at the end of nebulising the liquid solution "L" (that is, after reaching the threshold value) a predetermined quantity of a liquid substance "F" is in turn nebulised in the room, different from the liquid solution "L" and having a predetermined fragrance.

Preferably, there is a predetermined period of time between the end of the nebulising and the dispensing of the fragrance, more preferably fixed on the basis of operating protocols with the structure involved and in compliance with the national laws and regulations.

Thus, the liquid substance "F" is nebulised at the end of a further predetermined interval of time after completion of the nebulising of the liquid solution "L".

Advantageously, this allows an immediate sensorial check to be made that the treatment in the room "R" has been performed.

Preferably, the method also comprises a step of emitting an audible signal and/or a luminous signal at the end of the nebulising of the liquid substance "F".

In the preferred embodiment, once the diffusion or nebulising of the fragrance "F" (that is, of the liquid substance) has been completed, an intermittent audio signal is activated (generated by a siren) representing the status of the treatment completed.

Advantageously, even without hearing the audible signal, the operator can re-enter the room and immediately determine the positive conclusion of the treatment, by noting the scent of the fragrance "F" present in the air of the room "R".

Preferably, the predetermined quantity of liquid substance "F", that is, fragrance, nebulised in the room "R" is proportional to the cubic volume of the treated room.

In the embodiment illustrated, the liquid substance "F" is stored in a container 17 housed inside the diffuser device 1.

It should also be noted that the predetermined quantity of liquid substance "F" (that is, fragrance) is calculated as a function of the cubic volume of the room "R" to be treated.

Preferably, the method also comprises a step of controlling the consumption of liquid substance "F" by controlling the variation in weight of the container 17, in a similar manner to that for tank 2.

The invention achieves the proposed aims and brings significant advantages.

In effect, the use of a diffuser device interfaced with an electronic plate provided with all the identification data of the room allows an automatic and secure solution to be obtained for the traceability which allows the user to check, and therefore demonstrate, that clearly identified rooms have been sanitised with precise dates, methods and times.

It should be noted that the presence of the sensor means and of the control unit especially programmed can guarantee to the user that exactly the quantity of solution required to sanitise a given room has been dispensed.

Moreover, the use of a rewritable electronic label guarantees the identity and the authenticity of the solution used, avoiding tampering or falsifying of the solution.

Moreover, the preparation of a container with a liquid substance, having a predetermined fragrance, allows a method to be implemented which allows the user to check the disinfection with the sensors provided.

**Claims**

1. A diffuser device (1) for a system for the disinfection of spaces and surfaces, comprising:

   - a tank (2) containing a liquid solution (L),
   - a container (17) of a liquid substance (F), having a predetermined fragrance different from the liquid solution (L);
   - nebulising means (3) in fluid connection with the tank (2) and the container (17) and designed for drawing out the liquid solution (L) from the tank (2) and the liquid substance (F) from the container (17) spraying them in the space;
   - at least one control unit (8) programmed for controlling the nebulising means (3) in such a way as to draw out selectively from the tank (2) or from the container (17) as a function, respectively, of a first signal representing the start of a sanitisation and of a second signal representing the end of the nebulising, **characterised in that** the control unit (8) is programmed for measuring a predetermined interval of time between the end of nebulising and dispensing the liquid substance (F) so as to allow the complete removal of the bacteria by the sanitising liquid solution (L) nebulised during the treatment.

2. The diffuser device (1) according to claim 1, **characterised in that** it comprises sensor means (12) associated with the tank (2) and the control unit (8) and designed for measuring a quantity correlated with the quantity of liquid solution (L) dispensed by the nebulising means (3) and for sending a signal representing the quantity to the control unit (8); the control unit (8) being programmed for issuing a command to the nebulising means (3) for drawing out the liquid substance (F) from the container (17) after receiving, from the sensor means (12), a signal indicating that the threshold value of the quantity of liquid solution (L) to be dispensed has been exceeded.

3. The diffuser device (1) according to claim 2, **characterised in that** control unit (8) is designed for calculating the threshold value of the quantity of liquid solution (L) to be dispensed as a function of the cubic volume of the room (R) to be treated.

4. The diffuser device according to claim 2 or 3, **characterised in that** the sensor means (12) comprise at least one weight sensor (13) associated with the tank (2); the control unit (8) being designed for calculating a variation in the quantity of liquid solution (L) in the tank (2) as a function of a variation of the weight of the tank (2).

5. The diffuser device according to claim 4, **characterised in that** the sensor means (12) comprise at least one further weight sensor (19) associated with the container (17); the control unit (8) being designed for calculating a variation in the quantity of liquid substance (F) in the container (17) in as a function of a variation of the weight of the container (17).

6. The diffuser device according to any one of the preceding claims, **characterised in that** the control unit (8) is designed for calculating a predetermined quantity of liquid substance (F) to be dispensed as a function of a cubic volume of the room (R) to be treated.

7. A method for the disinfection of spaces and surfaces, comprising the steps of:

   - preparing a diffuser device (1) equipped with a tank (2) containing a liquid solution (L) and nebulising means (3) associated with the tank (2) and designed for drawing out the liquid solution (L) from the tank spraying it in the space;
   - nebulising in the room a predetermined quantity of liquid solution (L) for an interval of time;
   - nebulising in the room a predetermined quantity of a liquid substance (F), different from the liquid solution (L) and having a predetermined fragrance, after the interval of time, **characterised in that** the step of nebulising the liquid substance (F) is performed at the end of a further predetermined interval of time after the interval of time in which the predetermined quantity of liquid solution (L) is nebulised in the room (R), so as to allow the complete removal of the bacteria by the sanitising liquid solution (L) nebulised during the treatment.

8. The method according to claim 7, **characterised in that** the liquid substance (F) is stored in a container (17) housed inside the diffuser device (1).

9. The method according to claim 7 or 8, **characterised in that** the predetermined quantity of liquid substance (F) is calculated as a function of the cubic volume of the room (R) to be treated.

10. The method according to any one of claims 7 to 9, **characterised in that** it comprises a step of controlling the consumption of liquid substance (F) by controlling the variation in weight of the container (17) of liquid substance (F) during the nebulising.

**Patentansprüche**

1. Diffusorvorrichtung (1) für ein System zur Desinfektion von Räumen und Oberflächen, umfassend:

   - einen Tank (2), enthaltend eine flüssige Lösung (L);
   - einen Behälter (17) einer flüssigen Substanz (F), aufweisend einen vorgegebenen Duftstoff, der sich von der flüssigen Lösung (L) unterscheidet;
   - Vernebelungsmittel (3) in Fluidverbindung mit dem Tank (2) und dem Behälter (17) und ausgestaltet, um die flüssige Lösung (L) aus dem Tank (2) und die flüssige Substanz (F) aus dem Behälter (17) zu saugen und in den Raum zu sprühen;
   - mindestens eine Steuereinheit (8), die programmiert ist, um die Vernebelungsmittel (3) so zu steuern, dass selektiv aus dem Tank (2) oder aus dem Behälter (17) gesaugt wird, jeweils abhängig von einem ersten Signal, das den Start einer Keimfreimachung darstellt, bzw. von einem zweiten Signal, das das Ende der Vernebelung darstellt, **dadurch gekennzeichnet, dass** die Steuereinheit (8) programmiert ist, um ein vorgegebenes Zeitintervall zwischen dem Ende der Vernebelung und der Ausgabe der flüssigen Substanz (F) zu messen, sodass die komplette Beseitigung von Bakterien durch die während der Behandlung vernebelte keimfrei machende flüssige Lösung (L) ermöglicht wird.

2. Diffusorvorrichtung (1) nach Anspruch 1, **dadurch gekennzeichnet, dass** sie Sensormittel (12) umfasst, die mit dem Tank (2) und der Steuereinheit (8) verbunden und ausgestaltet sind, um eine Menge, die mit der Menge der flüssigen Lösung (L), die von den Vernebelungsmitteln (3) ausgegeben wird, korreliert ist, zu messen, und um ein die Menge darstellendes Signal an die Steuereinheit (8) zu senden, wobei die Steuereinheit (8) programmiert ist, um einen Befehl an die Vernebelungsmittel (3) zu übermitteln, um die flüssige Substanz (F) aus dem Behälter (17) zu saugen, nachdem von den Sensormitteln (12) ein Signal empfangen wurde, das angibt, dass der Schwellenwert der Menge der auszugebenden flüssigen Lösung (L) überschritten wurde.

3. Diffusorvorrichtung (1) nach Anspruch 2, **dadurch gekennzeichnet, dass** die Steuereinheit (8) ausgestaltet ist, um den Schwellenwert der Menge an auszugebender flüssiger Lösung (L) abhängig vom Kubikinhalt des zu behandelnden Raums (R) zu berechnen.

4. Diffusorvorrichtung nach Anspruch 2 oder 3, **dadurch gekennzeichnet, dass** die Sensormittel (12) mindestens einen Wägesensor (13) umfassen, der mit dem Tank (2) verbunden ist, wobei die Steuereinheit (8) ausgestaltet ist, um eine Veränderung der Menge der flüssigen Lösung (L) im Tank (2) abhängig von einer Gewichtsveränderung des Tanks (2) zu berechnen.

5. Diffusorvorrichtung nach Anspruch 4, **dadurch gekennzeichnet, dass** die Sensormittel (12) mindestens einen weiteren Wägesensor (19) umfassen, der mit dem Behälter (17) verbunden ist, wobei die Steuereinheit (8) ausgestaltet ist, um eine Veränderung der Menge der flüssigen Substanz (F) im Behälter (17) abhängig von einer Gewichtsveränderung des Behälters (17) zu berechnen.

6. Diffusorvorrichtung nach einem der vorhergehenden Ansprüche, **dadurch gekennzeichnet, dass** die Steuereinheit (8) ausgestaltet ist, um eine vorgegebene Menge an auszugebender flüssiger Substanz (F) abhängig von einem Kubikinhalt des zu behandelnden Raums (R) zu berechnen.

7. Verfahren zur Desinfektion von Räumen und Oberflächen, umfassend die folgenden Schritte:

    - Vorbereiten einer Diffusorvorrichtung (1), ausgestattet mit einem Tank (2), enthaltend eine flüssige Lösung (L), und Vernebelungsmitteln (3), verbunden mit dem Tank (2) und ausgestaltet, um die flüssige Lösung (L) aus dem Tank zu saugen und in den Raum zu sprühen;
    - Vernebeln einer vorgegebenen Menge an flüssiger Lösung (L) im Raum für ein Zeitintervall;
    - Vernebeln einer vorgegebenen Menge einer flüssigen Substanz (F) im Raum, die sich von der flüssigen Lösung (L) unterscheidet und einen vorgegebenen Duftstoff aufweist, nach einem Zeitintervall, **dadurch gekennzeichnet, dass** der Schritt zum Vernebeln der flüssigen Substanz (F) am Ende eines weiteren vorgegebenen Zeitintervalls nach dem Zeitintervall, in dem die vorgegebene Menge an flüssiger Lösung (L) im Raum (R) vernebelt wird, stattfindet, sodass die komplette Beseitigung der Bakterien durch eine während der Behandlung vernebelte keimfrei machende flüssige Lösung (L) ermöglicht wird.

8. Verfahren nach Anspruch 7, **dadurch gekennzeichnet, dass** die flüssige Substanz (F) in einem Behälter (17) gespeichert ist, der in der Diffusorvorrichtung (1) untergebracht ist.

9. Verfahren nach Anspruch 7 oder 8, **dadurch gekennzeichnet, dass** die vorgegebene Menge an flüssiger Substanz (F) abhängig vom Kubikinhalt des zu behandelnden Raums (R) berechnet wird.

10. Verfahren nach einem der Ansprüche 7 bis 9, **dadurch gekennzeichnet, dass** es einen Schritt zum Regeln des Verbrauchs an flüssiger Substanz (F) durch die Kontrolle der Gewichtsveränderung des Behälters (17) an flüssiger Substanz (F) während der Vernebelung umfasst.

**Revendications**

1. Dispositif diffuseur (1) pour un système de désinfection des espaces et des surfaces, comprenant :

    - un réservoir (2) contenant une solution liquide (L),
    - un récipient (17) d'une substance liquide (F), ayant une fragrance prédéterminée différente de la solution liquide (L) ;
- des moyens de nébulisation (3) en raccordement fluidique avec le réservoir (2) et le récipient (17) et conçus pour extraire la solution liquide (L) du réservoir (2) et la substance liquide (F) du récipient (17) en les pulvérisant dans l'espace ;
- au moins une unité de commande (8) programmée pour commander les moyens de nébulisation (3) de manière à extraire de façon sélective à partir du réservoir (2) ou à partir du récipient (17) en fonction, respectivement, d'un premier signal représentatif du démarrage d'une désinfection et d'un second signal représentatif de la fin de la nébulisation, **caractérisé en ce que** l'unité de commande (8) est programmée pour mesurer un intervalle de temps prédéterminé entre la fin de la nébulisation et la distribution de la substance liquide (F) de manière à permettre l'élimination totale des bactéries par la solution liquide désinfectante (L) nébulisée lors du traitement.

2. Dispositif diffuseur (1) selon la revendication 1,
**caractérisé en ce qu'**il comprend des moyens de détection (12) associés au réservoir (2) et à l'unité de commande (8) et conçus pour mesurer une quantité correspondant à la quantité de solution liquide (L) distribuée par les moyens de nébulisation (3) et pour envoyer un signal, représentatif de la quantité, à l'unité de commande (8) ; l'unité de commande (8) étant programmée pour délivrer une commande aux moyens de nébulisation (3) pour extraire la substance liquide (F) du récipient (17) après réception, à partir des moyens de détection (12), d'un signal indiquant que la valeur seuil de la quantité de solution liquide (L) à distribuer a été dépassée.

3. Dispositif diffuseur (1) selon la revendication 2,
**caractérisé en ce que** l'unité de commande (8) est conçue pour calculer la valeur seuil de la quantité de solution liquide (L) à distribuer en fonction du volume cubique du local (R) à traiter.

4. Dispositif diffuseur selon la revendication 2 ou 3,
**caractérisé en ce que** les moyens de détection (12) comprennent au moins un détecteur de poids (13) associé au réservoir (2) ; l'unité de commande (8) étant conçue pour calculer une variation de la quantité de solution liquide (L) dans le réservoir (2) en fonction d'une variation du poids du réservoir (2).

5. Dispositif diffuseur selon la revendication 4,
**caractérisé en ce que** les moyens de détection (12) comprennent au moins un détecteur de poids (19) supplémentaire associé au réservoir (17) ; l'unité de commande (8) étant conçue pour calculer une variation de la quantité de substance liquide (F) dans le récipient (17) en fonction d'une variation du poids du récipient (17).

6. Dispositif diffuseur selon l'une quelconque des revendications précédentes, **caractérisé en ce que** l'unité de commande (8) est conçue pour calculer une quantité prédéterminée de substance liquide (F) à distribuer en fonction d'un volume cubique du local (R) à traiter.

7. Procédé de désinfection d'espaces et de surfaces, comprenant les étapes de :

- préparer un dispositif diffuseur (1) équipé d'un réservoir (2) contenant une solution liquide (L) et des moyens de nébulisation (3) associés au réservoir (2) et conçus pour extraire la solution liquide (L) du réservoir en la vaporisant dans l'espace ;
- nébuliser dans le local une quantité prédéterminée de solution liquide (L) pendant un intervalle de temps ;
- nébuliser dans le local une quantité prédéterminée d'une substance liquide (F), différente de la solution liquide (L) et ayant une fragrance prédéterminée, après l'intervalle de temps, **caractérisé en ce que** l'étape consistant à nébuliser la substance liquide (F) est réalisée à la fin d'un intervalle de temps supplémentaire prédéterminé après l'intervalle de temps au cours duquel la quantité prédéterminée de solution liquide (L) est nébulisée dans le local (R), de manière à permettre l'élimination totale des bactéries par la solution liquide désinfectante (L) nébulisée pendant le traitement.

8. Procédé selon la revendication 7, **caractérisé en ce que** la substance liquide (F) est stockée dans un récipient (17) logé à l'intérieur du dispositif diffuseur (1).

9. Procédé selon la revendication 7 ou 8, **caractérisé en ce que** la quantité prédéterminée de substance liquide (F) est calculée en fonction du volume cubique du local (R) à traiter.

10. Procédé selon l'une quelconque des revendications de 7 à 9, **caractérisé en ce qu'**il comprend une étape consistant à contrôler la consommation de substance liquide (F) en contrôlant la variation du poids du récipient (17) de substance liquide (F) lors de la nébulisation.

FIG. 1

FIG. 2

EP 2 991 690 B1

FIG.3

FIG.4

FIG.5a

FIG.5b

FIG.5c

FIG.6

**REFERENCES CITED IN THE DESCRIPTION**

**Patent documents cited in the description**

- US 2010326117 A

- WO 0050320 A